# EUROPEAN PATENT SPECIFICATION

(11) **EP 2 714 691 B1**
(45) Date of publication and mention of the grant of the patent: **07.09.2016**
(21) Application number: 11727115.5
(22) Date of filing: 31.05.2011
(51) Int. Cl.: C07D 473/18

(54) **PROCESS FOR THE PREPARATION OF 2-AMINO-9-((2-PHENYL-1,3-DIOXAN-5-YLOXY)METHYL)-1H-PURIN-6(9H)-ONE COMPOUND USEFUL IN THE PREPARATION OF VALGANCICLOVIR**
VERFAHREN ZUR HERSTELLUNG VON 2-AMINO-9- ((2-PHENYL-1,3-DIOXAN-5-YLOXY)METHYL) (-1H-PURIN-6(9H) -ON-VERBINDUNG ZUR HERSTELLUNG VON VALGANCICLOVIR
PROCÉDÉ DE PRÉPARATION D'UN COMPOSÉ DE 2-AMINO-9-((2-PHÉNYL-1,3-DIOXAN- 5-YLOXY)-MÉTHYL)-1H-PURIN-6(9H)-ONE UTILE DANS LA PRÉPARATION DU VALGANCICLOVIR

(43) Date of publication of application: 09.04.2014
(73) Proprietor: Pharmathen S.A., 15351 Pallini Attikis (GR)
(72) Inventor: KOFTIS, Theocharis, V., GR-546 43 Thessaloniki (GR); GEORGOPOULOU, Ioanna, GR-542 48 Thessaloniki (GR); VARVOGLI, Anastasia, Aikaterini, GR-551 33 Thessaloniki (GR); ZITROU, Asteria, GR-546 22 Thessaloniki (GR)
(86) International application number: PCT/EP2011/002684
(87) International publication number: WO 2012/163373

(56) References cited:
- EP-A2- 0 694 547
- WO-A1-97/27197

## Description

### TECHNICAL FIELD OF THE INVENTION

The present invention relates to a process for the preparation of 2-amino-9-((2-phenyl-1,3-dioxan-5-yloxy)methyl)-1*H*-purin-6(9*H*)-one, which is an intermediate compound useful in the process for the preparation of Valganciclovir, and novel intermediates compound obtained thereof.

### BACKGROUND OF THE INVENTION

Valganciclovir is a prodrug of 2-amino-9-((1,3-dihydroxypropan-2-yloxy)methyl)-1*H-*purin-6(9*H*)-one commonly known as Ganciclovir, said prodrug is known to have antiviral properties used to treat virus infections especially cytomegalovirus infections.

Valganciclovir, which is the L-valine ester of Ganciclovir, is chemically designated as (2S)-2-((2-amino-6-oxo-1,6-dihydropurin-9-yl)methoxy)-3-hydroxypropyl 2-amino-3-methylbutanoate and is presented by the chemical structure of Formula I.

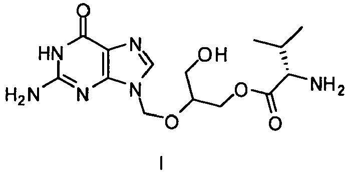

Ganciclovir is used mostly as an intravenous infusion as it has a very low rate of absorption when administered orally, whereas Valganciclovir is rapidly converted to Ganciclovir when administered orally, by intestinal and hepatic esterases.

Various methods for the preparation of Valganciclovir are already known.

EP-B-0877746 discloses the esterification of optionally amino-protected Ganciclovir with an L-valine derivative to give the protected bis-valine ester of Ganciclovir (step a, scheme 1), followed by cleavage of one of the ester moieties (step b, scheme 1) with n-propylamine in hexane. The preparation of the protected mono-L-valine ester proceeds with moderate conversion, resulting in a mixture of the bis- and mono- valine esters as well as the free diol (Ganciclovir). However, no method for the separation of this mixture is mentioned and a possible use of column chromatography would severely compromise the industrial applicability of the described process.

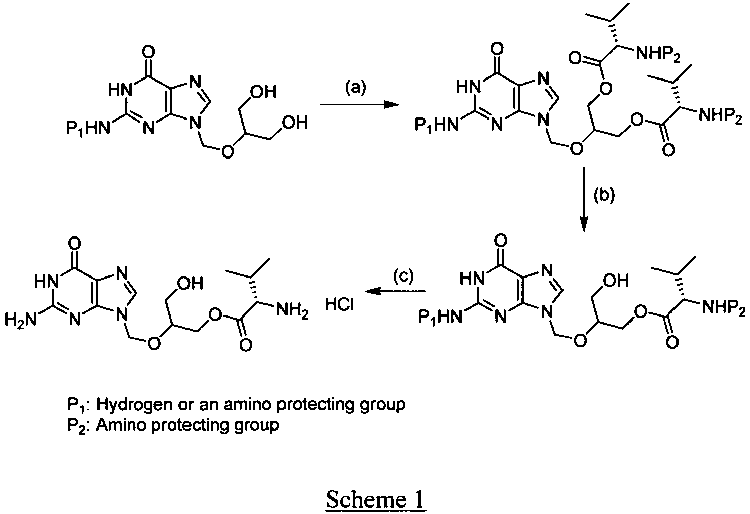

In EP-B-0885224 an improved process for the preparation of Valganciclovir is described, wherein a monocarboxylate intermediate (step b, scheme 2) is prepared through a cyclic orthoester of Ganciclovir (step a, scheme 2). Subsequently monoesterification by an activated L-valine derivative provides the monocarboxylate-monovalinate (step c, scheme 2), which may be selectively hydrolyzed to the mono-L-valine ester of Ganciclovir (step d, scheme 2). Although said process is considered advantageous, the intermediates obtained according to said process are contaminated with significant amounts of Ganciclovir, bis-z-valine or bis-valine ester of Ganciclovir and hence, additional purification steps are required for the process to be rendered efficient in large scale application.

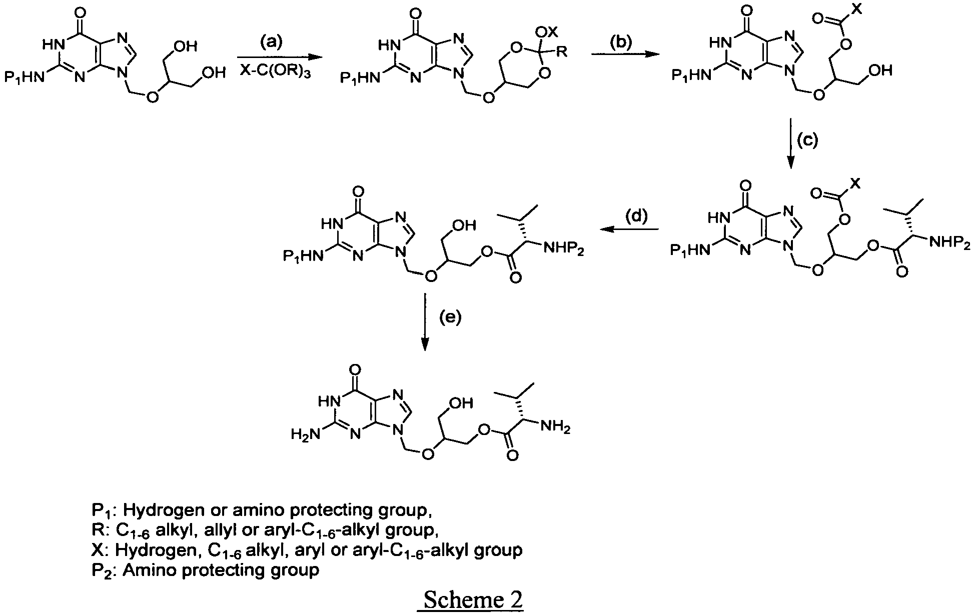

Further, EP-B-0694547 discloses Valganciclovir in the form of its (R) or (*S*) diastereomers or mixtures of the two. A selective process for the preparation of Valganciclovir, or a pharmaceutically acceptable salt thereof, is disclosed, which involves protection of either one or both hydroxy groups of optionally amino-protected Ganciclovir.

Treating 2-amino-9-((1-(benzyloxy)-3-hydroxypropan-2-yloxy)methyl)-1*H*-purin-6(9*H*)-one with an activated L-valine provides (2*S*)-2-((2-amino-6-oxo-1*H*-purin-9(6*H*)-yl)methoxy)-3-(benzyloxy)propyl)-2-(benzyloxycarbonylamino)-3-methylbutanoate, which is then deprotected to give Valganciclovir in crystalline form.

In said process, a novel mono-benzyl protected Ganciclovir intermediate is used (step b, scheme 3) which, upon conversion to the respective ester of protected L-valine (step c, scheme 3), may be subsequently reduced to provide Valganciclovir. However, the deprotection of dibenzyl Ganciclovir (step b, scheme 3) is not chemoselective and the desired monobenzyl intermediate has to be separated by column chromatography from the un-reacted dibenzyl-compound and possibly the over-reacted diol (Ganciclovir). As a result, the total yield of the stage is moderate and the process to obtain 2-amino-9-((1-(benzyloxy)-3-hydroxypropan-2-yloxy)methyl)-1*H*-purin-6(9*H*)-one does not offer any additional benefits for industrial use regarding purification procedures and overall cost.

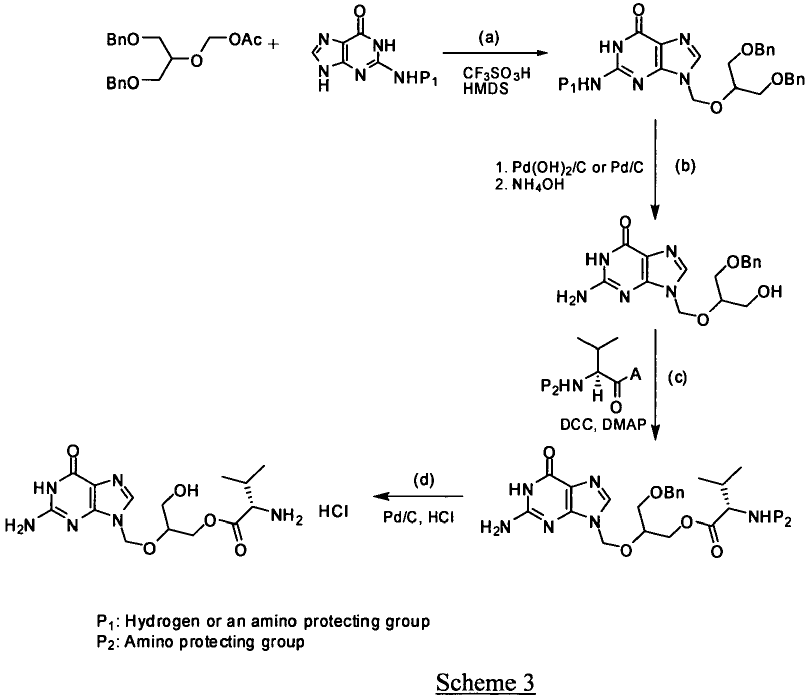

Although each of the above patents represent an attempt to overcome the disadvantages in the prior art processes, there still exists a need for a cost-effective and safer process for large scale production of Valganciclovir, which provides Valganciclovir in higher yield by controlling the overall quality.

### SUMMARY OF THE INVENTION

It is, therefore, an object of the present invention to provide an improved process for the preparation of 2-amino-9-((2-phenyl-1,3-dioxan-5-yloxy)methyl)-1*H*-purin-6(9*H*)-one of Formula II, as a key intermediate useful in the preparation of Valganciclovir, which overcomes the deficiencies of the prior art processes and results to a cost effective industrial production without sacrificing the yield and quality of the product.

Another object of the present invention is to provide a process for the preparation of Valganciclovir or pharmaceutically acceptable salts thereof by using novel compound of Formula II.

Further object of the present invention is to provide a novel intermediate compound, 2-amino-9-((2-phenyl-1,3-dioxan-5-yloxy)methyl)-1*H*-purin-6(9*H*)-one which is stable under intense alkaline conditions, yet sufficiently reactive to provide the key products in high yield and purity, therefore efficient when used in the process for the preparation of Valganciclovir.

A further object of the present invention is esterification of compound of Formula III with *N*-carbobenzyloxy-L-valine (*N*-CBZ- L-valine) of Formula IV to afford compound of Formula V in on organic solvent which is dimethylsulfoxide (DMSO).

In accordance with the above objects of the present invention, a process for the preparation of 2-amino-9-((1-(benzyloxy)-3-hydroxypropan-2-yloxy)methyl)-1*H*-purin-6(9*H*)-one of Formula III is provided according to the following steps:
a) Treating of Ganciclovir with benzaldehyde or benzaldehyde dimethyl acetal to obtain 2-amino-9-((2-phenyl-1,3-dioxan-5-yloxy)methyl)-1*H*-purin-6(9*H*)-one of Formula 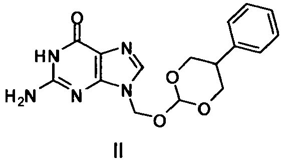
b) Reductive cleavage of compound of Formula II with a reducing agent such as sodium borohydride and a Lewis acid, such as aluminum chloride, to obtain 2-amino-9-((1-(benzyloxy)-3-hydroxypropan-2-yloxy)methyl)-1*H*-purin-6(9*H*)-one of Formula III

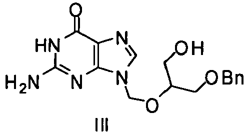

Another object of the present invention is to provide compound of Fomula II defined in claim 10.

Preferred embodiments of the present invention are set out in dependent claims 2-9.

Other objects and advantages of the present invention will become apparent to those skilled in the art in view of the following detailed description.

### DETAILED DESCRIPTION OF THE PRESENT INVENTION

The present invention relates to a novel process for the preparation of Formula II, which is an intermediate compound useful for the preparation of Valganciclovir.

According to the present invention, the process for the preparation of 2-amino-9-((1-(benzyloxy)-3-hydroxypropan-2-yloxy)methyl)-1*H*-purin-6(9*H*)-one of Formula III comprises the following steps:

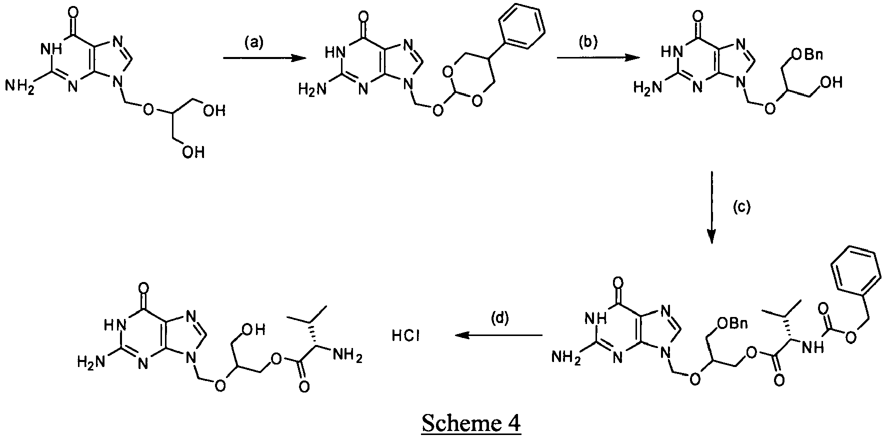

### Step a) of scheme 4: Preparation of compound of Formula II

Ganciclovir is added to a polar aprotic solvent, such as dimethylformamide (DMF), dimethylacetamide or DMSO, preferably DMSO. The reaction mass is heated and an efficient reagent for 1,3-benzylidene acetal formation is added to the solution, such as benzaldehyde or benzaldehyde dimethyl acetal, preferably benzaldehyde dimethyl acetal. The reaction is carried out in the presence of a catalytic amount of an acid such as p-toluenesulfonic acid, sulfuric acid, camphorosulphonic acid, zinc chloride, tin chloride, etc. Preferred acid is p-toluenesulfonic acid. The reaction is heated at temperature from about 50° to about 100 °C, preferably at 70°-80°C, for 10-24 hours, preferably for 12-18 hours. Upon reaction completion, adjustment to neutral or mild basic conditions is required, at reaction temperature. Preferable pH value is from 7.5 to 8.5 using sodium hydroxide, ammonium hydroxide, triethylamine, preferably triethylamine. The reaction mass is then cooled to ambient temperature and diluted with ethyl acetate (about 1-3 volumes, preferably 1 volume). Precipitation of the product is effected by dropwise addition of D.M. water from 10 to 20 volumes, preferably 15 volumes. Excess benzaldehyde is washed away with 3-10 volumes of toluene, at temperature from about 25° to about 70°C, preferably with 5 volumes at 55-65°C. Crude product is purified by recrystallisation in D.M. water or a mixture of D.M. water and organic solvent such as methanol, ethanol, dichloromethane, preferably ethanol, under alkaline conditions. The pH of the above solution is adjusted to value from 9 to 11, preferably pH value 10 to11 with aqueous HCl 3N, and the compound of Formula II is collected by filtration and dried at about 75-85°C.

### Step b) of scheme 4: Reductive cleavage of compound of Formula II

The product of step (a) is added in a mixture of aluminum chloride (AlCl₃) and sodium borohydride (NaBH₄) in an inert organic solvent, such as tetrahydrofuran (THF) or mixture of THF and dichloromethane (DCM). The molar ratio for aluminum chloride and sodium borohydride with respect to the starting material is 3:1 to 8:1, preferably 4:1 to 7:1 for each reagent. Small amounts of water are periodically added to the reaction mixture until conversion to 2-amino-9-((1-(benzyloxy)-3-hydroxypropan-2-yloxy)methyl)-1*H*-purin-6(9*H*)-one. The reaction is carried out at temperature from about 0° to about 10°C, preferably at temperature from about 2° to about 8°C.

After completion, the reaction mass is quenched with methanol and D.M. water, most preferably with D.M. water. The precipitated reaction mass is collected by filtration. The crude mass is purified by recrystallisation in D.M. water or a mixture of D.M. water and organic solvent such as methanol, ethanol, DCM, preferably ethanol, under alkaline conditions. The pH of the above solution is adjusted to values from 9 to11, preferably from 10 to 11, with aqueous HCl 3N and the product of Formula III is collected by filtration and dried under vacuum at temperature from about 75°C to about 85°C.

### Step c) of scheme 4: Esterification of compound of Formula III.

2-amino-9-((1-(benzyloxy)-3-hydroxypropan-2-yloxy)methyl)-1*H*-purin-6(9*H*)-one obtained in previous step is esterified with 3 equivalents of *N*-CBZ-L-valine and 1.2 equivalent of a suitable coupling agent or dehydrating agent, most preferably 1,3-dicyclohexyl carbodiimide. The protected amino acid is dissolved in an inert solvent, such as halogenated lower alkane, under inert atmosphere, and the coupling agent is added. The reaction mixture is stirred at temperature from about 25°C to about 35°C. The reaction mass is filtered and the anhydride of the protected amino acid is isolated. Amount corresponding to 1 equivalent of the product of step b, dissolved in DMF or DMSO, is added to the anhydride. The present inventors have found that compound of Formula III has better solubility when DMSO is used instead of DMF, and the reaction reaches completion significantly faster. The mixture is placed under inert atmosphere, and the reaction is carried out at temperature from about 25° to about 35°C for 4-72 hours until completion. The isolation and purification of the product is carried out by dilution in water to precipitate or other conventional techniques, such as extraction with an organic solvent or mixture of organic solvents, and precipitation of (2*S*)-2-((2-amino-6-oxo-1*H*-purin-9(6*H*)-yl)methoxy)-3-(benzyloxy)propyl)-2-(benzyloxycarbonylamino)-3-methylbutanoate.

### Step d) of scheme 4: Final deprotection of compound of Formula V to give Valganciclovir HCl

The protected mono-valine ester of Ganciclovir of Formula V, is deprotected by hydrogenolysis in the presence of palladium on carbon catalyst in methanol, under hydrogen gas in a pressurized vessel at temperature from about 25° to 100°C. After completion of the reaction the catalyst is filtered and washed, and the combined filtrates are concentrated under reduced pressure to remove solvent from Valganciclovir hydrochloride salt. Isolation of the amorphous Valganciclovir hydrochloride can be directly realized by spray drying techniques of an aqueous solution of the product, optionally using a cosolvent, such as ethanol, isopropanol or methanol, preferably isopropanol.

Alternatively, Valganciclovir hydrochloride can be obtained by a) concentration under vacuum to remove the solvent b) azeotropical water removal c) treatment with an organic solvent.

The process of the present invention will be demonstrated in more details with reference to the following examples, which are provided by way of illustration only and should not be construed as limit to the scope of the reaction in any manner.

### EXAMPLES

### Example 1: Preparation of 2-amino-9-((2-phenyl-1,3-dioxan-5-yloxy)methyl)-1H-purin-6(9H)-one

In a 21t R.B.flask is charged 500ml DMSO and 100g 2-amino-9-((1,3-dihydroxypropan-2-yloxy)methyl)-1*H*-purin-6(9*H*)-one (0.392mol) at temperature of about 25 - 35°C, under stirring. The reaction mass is heated at temperature from about 70° to about 80°C until all material dissolves. In the above solution is slowly added 129 ml benzaldehyde dimethyl acetal (0.862mol) and 2.6g p-toluenesulfonic acid monohydrate (0.014mol). The reaction mass is stirred at temperature from about 70 to 80 °C for about 12-18 hours. Upon completion of the reaction, 20ml triethylamine is charged to the reaction mass and cooled to temperature from about 25 to about 35°C. At this temperature 100ml ethyl acetate is added under stirring, and 1500ml D.M. water within 90-150 minutes, keeping the temperature at 25-35°C (external cooling is used, if required). The reaction mass is stirred for additional 300-360 minutes. The precipitated solid is filtered off under reduced pressure and sucked dry in the vacuum pump for about 45-60 minutes. The wet cake is transferred into a flask and washed under stirring with 500ml toluene at temperature from about 45 to 55°C, for 120-180 minutes. The suspension is cooled to ambient temperature, filtered off under reduced pressure and sucked dry for 30-45 minutes. To the wet cake 100ml ethanol and 500ml D. M. water is added. To the above suspension 120-160ml aq. sodium hydroxide 20% w/w is slowly added under vigorous stirring, and further stirred for 30-60minutes. To the clear solution is added 100-150ml hydrochloric acid 3N till adjustment to pH 10-11. The suspension is stirred for additional 60-120minutes at ambient temperature. The precipitated solid is filtered off under reduced pressure and sucked dry in the vacuum pump for 45-60 minutes. The wet solid is washed with 800 ml D.M. water for 60-90 minutes. The solid is filtered again under reduced pressure and sucked dry for 45-60 minutes. The wet cake is unloaded and dried in a vacuum oven at temperature from about 75°C to about 85°C. Weight of 2-amino-9-((2-phenyl-1,3-dioxan-5-yloxy)methyl)-1*H*-purin-6(9*H*)-one is 95-105g (71-78% yield).

### Analysis of the solid obtained showed:

Desired product (benzylidene acetal of Ganciclovir) about 95%
Ganciclovir about 0.4%
**mp:** 254.2-254.6°C;
**LC-MS** (CI) m/z: 344 (MH⁺);
**IR** (KBr) cm⁻¹ 3455, 3321, 3184, 2858, 2724, 1724, 1690, 1630, 1487, 1389, 1274, 1220, 1182, 1145, 1098, 1028, 755, 698;
**¹H NMR** (300 MHz, DMSO-D6) *δ* 10.75 (br,1H), 7.92-7.87 (2s,1H), 7.39-7.31 (m, 5H), 6.58-6.54 (2s,2H), 5.55-5.42 (m, 3H), 4.13-3.48 (m, 5H);
**¹³C NMR** (75 MHz, DMSO-D6) *δ* 156.8-156.7, 153.9-153.8, 151.4-151.2, 138.4-137.6, 137.6-137.4, 128.6-128.4, 127.9-127.8, 126-125.8, 116.5-116.3, 100.1-99.9, 70.8-69.7, 68.9-68.5, 68.3-66.8.
wherein:
¹H NMR: proton nuclear magnetic resonance spectroscopy
¹³C NMR: carbon nuclear magnetic resonance spectroscopy
IR: infrared spectroscopy
δ: the chemical shift referenced against tetramethylsilane (TMS)
s, d, t and m are referred to singlet, doublet, triplet and multiplet peaks respectively

### Example 2: Preparation of 2-amino-9-((1-(benzyloxy)-3-hydroxypropan-2-yloxy) methyl)-1H-purin-6(9H)-one

In a 51t R.B.flask is added 1500ml THF and 1500ml DCM at temperature from about 25° to about 30°C under stirring, and cooled at temperature from about 0°C to about 10°C. To the mix of solvents is added 272g aluminum chloride (1.893mol), keeping the temperature from about 0° to 10°C. Sodium borohydride 77g (1.893 mol) is added, followed by addition of 100g 2-amino-9-((2-phenyl-1,3-dioxan-5-yloxy) methyl)-1*H*-purin-6(9*H*)-one (0.291ml). Bubbler is adjusted, and 6-8ml D.M. water is added. The reaction mass is stirred for about 30 to 60 minutes, at the same temperature, and the progress is checked by TLC (CHCl₃: MeOH 60:10). Addition of 4-6ml D.M.water is repeated until completion. The reaction mixture is quenched by dropwise addition of 500ml D.M. water, keeping the temperature within 2° to 8°C. More 500ml D. M. water are added under stirring, allowing the reaction mass to warm to temperature 25-35°C. The precipitated solid is filtered under reduced pressure. To the wet cake is charged 500ml D. M water, 100ml ethanol and 100-150ml aq. sodium hydroxide 20% w/w, under stirring, until clear solution. Hydrochloric acid 3N 100-150ml are added till adjustment to pH 10-11. The precipitated solid is filtered off under reduced pressure and sucked dry. The wet material is washed with 500 ml D.M. water for 60-90 minutes, filtered again under reduced pressure and sucked dry. The wet cake is unloaded and dried in a vacuum oven at 75-85°C. Approximately weight of 2-amino-9-((1-(benzyloxy)-3-hydroxypropan-2-yloxy) methyl)-1*H*-purin-6(9*H*)-one is 55-65g (55-65% yield).

Analysis of the solid obtained showed:
Desired product (mono-hydroxy protected ganciclovir) about 90%
Benzylidene acetal of Ganciclovir about 4%
Ganciclovir less than 0.1%
**LC-MS** (CI) m/z: 344 (MH⁺);
**IR** (KBr) cm⁻¹: 3420, 3320, 3159, 3101, 2942, 2892, 1687, 1658, 1491, 1475, 1367, 1064, 1045;
**¹H NMR** (300 MHz, DMSO-D6) *δ* 10.74 (br, 1H), 7.92 (s,1H), 7.32-7.22 (m,5H), 6.58 (s,2H), 5.48 (s,2H), 4.41 (s,2H), 3.82-3.79 (m, 2H), 3.48-3.37 (m, 4H);
**¹³C NMR** (75 MHz, DMSO-D6) *δ* 156.5, 153.9, 151.1, 138.3, 137.5, 128.1, 127.2, 127.2, 115.8, 78.4, 72.2, 71.7, 69.8, 60.9

### Example 3(a): Preparation of (2S)-2-((2-amino-6-oxo-1H-purin-9(6H)-yl)methoxy)-3-(benzyloxy)propyl-2-(benzyloxycarbonylamino)-3-methylbutanoate

To a solution of *N*-benzyloxycarbonyl-L-valine 218.5g (0.868mol) in 360ml DCM under nitrogen is added dicyclohexylcarbodiimide 71.7g (0.347mol).The mixture is stirred at room temperature for 20-30 hours. The resulting mixture is filtered to remove the insoluble material, washed with 360ml DCM and evaporated under reduced pressure. The foam residue is dissolved in 100ml DMF and added directly in a mixture of 100g 2-amino-9-((2-phenyl-1,3-dioxan-5-yloxy) methyl)-1*H*-purin-6(9*H*)-one (0.289mol) in 600ml DMF. 4-Dimethylaminopyridine 8.83g (0.072mol) is added and the mixture is stirred under nitrogen for 18-24 hours. The reaction mass is poured into 6000ml D.M. water and extracted with 1750ml ethyl acetate and 1750ml toluene. The aqueous layer is separated and the organic layer is washed with half saturated 1000ml sodium bicarbonate, followed by 1000ml D.M. water. The organic layer is dried over magnesium sulfate and concentrated under reduced pressure. The residue is precipitated from a mixture of ethyl acetate and cyclohexane to provide 120-130g of of (2*S*)-2-((2-amino-6-oxo-1*H*-purin-9(6*H*)-yl)methoxy)-3-(benzyloxy)propyl-2-(benzyloxycarbonylamino)-3-methylbutanoate as an amorphous solid (72-78% yield).

Purity (by HPLC):
CBZ-protected mono L-valine ester of Ganciclovir more than 97%
CBZ-protected bis L-valine ester of Ganciclovir less than 0.1%

### Example 3(b): Preparation of (2S)-2-((2-amino-6-oxo-1H-purin-9(6H)-yl)methoxy)-3-(benzyloxy)propyl-2-(benzyloxycarbonylamino)-3-methylbutanoate

To a solution of N-benzyloxycarbonyl-L-valine 218.5g (0.868mol) in 360ml DCM under nitrogen is added dicyclohexylcarbodiimide 71.7g (0.347mol).The mixture is stirred at room temperature for 20-30 hours. The resulting mixture is filtered to remove the insoluble material, washed with 360ml DCM and evaporated under reduced pressure. The foam residue is dissolved in 100ml DMSO and added directly in a mixture of 100g 2-(2-amino-1,6-dihydro-6-oxo-purin-9-yl)methoxy-3-benzyloxy-propan-1-ol (0.289mol) in 300ml DMSO. 4-Dimethylaminopyridine 8.83g (0.072mol) is added and the mixture is stirred under nitrogen for 4-6 hours, at which point TLC indicated consumption of the starting material. The reaction mass is poured into 61t D.M.water and extracted with 1500ml ethyl acetate and 1500ml toluene. The aqueous layer is separated and the organic layer is washed with half saturated 1000ml sodium bicarbonate, followed by 1000ml water. The organic layer is dried over magnesium sulfate and concentrated under reduced pressure. The residue is precipitated from a mixture of isopropanol and t-butyl methylether to provide 125-135g of (2*S*)-2-((2-amino-6-oxo-1*H*-purin-9(6*H*)-yl)methoxy)-3-(benzyloxy)propyl-2-(benzyloxycarbonylamino)-3-methylbutanoate as an amorphous solid (75-81% yield).

Purity (by HPLC):
CBZ-protected mono L-valine ester of Ganciclovir more than 97%
CBZ-protected bis L-valine ester of Ganciclovir less than 0.1%

### Example 4: Preparation of (2S)-2-((2-amino-6-oxo-1H-purin-9(6H)-yl)methoxy)-3-hydroxypropyl 2-amino-3-methylbutanoate hydrochloride

(2*S*)-2-((2-amino-6-oxo-1*H*-purin-9(6*H*)-yl)methoxy)-3-(benzyloxy)propyl-2-(benzyloxycarbonylamino)-3-methylbutanoate 100g (0.173mol) is dissolved in 900ml methanol and concentrated 17.4ml hydrochloric acid (0.173mol) is added dropwise. The mixture is placed under nitrogen and 60g 5% palladium on carbon, 50% wet, is added. The mixture is hydrogenated in a Parr bomb under hydrogen (100-150psi pressure) until completion. The mixture is filtered through a pad of Celite and the residue is washed with 300ml methanol. The filtrate is evaporated under reduced pressure at 25-35°C to dryness.

The residue is dissolved in 300ml D.M. water, filtered to remove the undissolved material and the cake is washed with 200ml D.M. water. Additional 500ml D.M. water are added. The clear solution is spray dried at 55-65°C, 6.0kg nitrogen pressure and at a rate of about 10ml/min. The material is recovered from the receiver, and dried at 40-45°C under vacuum for 6-8 hours to yield 50-60g of (2*S*)-2-((2-amino-6-oxo-1*H*-purin-9(6*H*)-yl)methoxy)-3-hydroxypropyl 2-amino-3-methylbutanoate hydrochloride as an amorphous material (74-88% yield).

Purity (by HPLC):
Valganciclovir HCl more than 97%
Bis L-valine ester of Ganciclovir less than 0.1%
Ganciclovir about 1%

It is apparent from the examples of the present invention that the use of 2-amino-9-((2-phenyl-1,3-dioxan-5-yloxy)methyl)-1*H*-purin-6(9*H*)-one as an intermediate compound substantially free from residual Ganciclovir, leads to a cost-effective, scalable and safe process for the preparation of Valganciclovir which makes it suitable for industrial application.

## Claims

1. A process for the preparation of Valganciclovir and pharmaceutically acceptable salts thereof, which comprises the following steps:
a) Treating Ganciclovir with benzaldehyde or benzaldehyde dimethyl acetal to obtain 2-amino-9-((2-phenyl-1,3-dioxan-5-yloxy)methyl)-1*H*-purin-6(9*H*)-one of Formula II 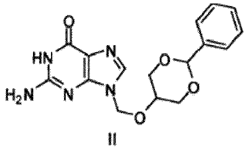
b) Reductive cleavage of compound of Formula II to obtain 2-amino-9-((1-(benzyloxy)-3-hydroxypropan-2-yloxy)methyl)-1*H*-purin-6(9*H*)-one of Formula III in an organic solvent.

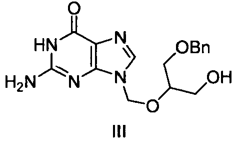

2. The process according to claim 1, wherein the reaction of Ganciclovir with benzaldehyde or benzaldehyde dimethyl acetal is conducted in a polar aprotic organic solvent and in the presence of an acid catalyst.

3. The process according to claim 2, wherein said polar aprotic organic solvent is DMF or DMSO, preferably DMSO.

4. The process according to claim 2, wherein said acid catalyst is selected from a group consist of p-toluenesulfonic acid, sulfuric acid, camphorsulphonic acid, zinc chloride, and tin chloride, preferably p-toluenesulfonic acid.

5. The process according to claim 1, wherein reductive cleavage of step b) is carried out in a polar organic solvent selected from THF and a mixture of THF and DCM.

6. The process according to claim 1, wherein reductive cleavage of compound of Formula II is carried out with NaBH₄/AlCl₃.

7. The process according to claim 6, wherein the molar ratio of AlCl₃ and NaBH₄ with respect to the starting material is 3:1 to 8:1, preferably 4:1 to 7:1 for each reagent.

8. The process according to claim 1, wherein it further comprises the steps:
c) Treating compound of Formula III with *N*-CBZ-L-valine of Formula IV, in an organic solvent and in the presence of a coupling agent or a dehydrating agent; 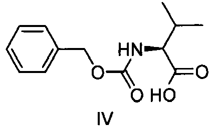
d) Deprotecting the amino-protected carbobenzyloxy group and hydroxy- protected benzyl group of the obtained compound of Formula V to obtain Valganciclovir; and 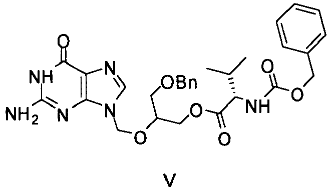
e) Optionally converting Valganciclovir into a pharmaceutically acceptable salt thereof.

9. The process according to claim 8, wherein the solvent used in step (c) is DMF or DMSO, preferably DMSO.

10. A compound of Formula II, which is chemically designated as 2-amino-9-((2-phenyl-1,3-dioxan-5-yloxy)methyl)-1*H*-purin-6(9*H*)-one.

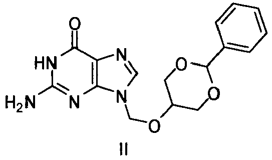

11. Use of compound of Formula II for the preparation of Valganciclovir or pharmaceutically acceptable salts thereof.

## Patentansprüche

1. Verfahren zur Herstellung von Valganciclovir und pharmazeutische annehmbare Salze davon, welches die folgenden Schritte umfaßt:
a) die Behandlung von Ganciclovir mit benzaldehyde oder benzaldehyddimethylacetal um 2-amino-9-((2-phenyl-1,3-dioxan-5-yloxy)methyl)-1H-purin-6(9H)-on der Formel II zu erhalten; 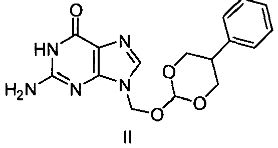
b) reduktive Spaltung des Verbindungs der Formel II um 2-amino-9-((1-(benzyloxy)-3 -hydroxypropan-2-yloxy)methyl)-1H-purin-6(9H)-one der Formel III in einem organischen Lösungsmittel zu erhalten.

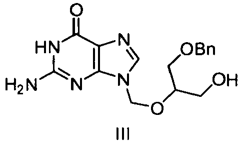

2. Verfahren nach Anspruch 1, wobei der Reaktion von Ganciclovir mit benzaldehyde order benzaldehyddimethylacetal in einem polaren aprotischen organischen Lösungsmittel und in Gegenwart eines Säurekatalysators durchgeführt wird.

3. Verfahren nach Anspruch 2, wobei das polaren aprotischen organischen Lösungsmittel DMF oder DMSO, vorzugsweise DMSO ist.

4. Verfahren nach Anspruch 2, wobei der Säurekatalysator aus einer Gruppe ausgewählt ist, der aus p-Toluolsulfonsäure, Schwefelsäure, Camphersulfonsäure, Zinkchlorid, und Titanchlorid, vorzugsweise p-Toluolsulfonsäure bestehend.

5. Verfahren nach Anspruch 1, wobei reduktive Spaltung von Schritt b) in einem polaren aprotischen organischen Lösungsmittel durchgeführt wird, das aus THF oder eine Mischung aus THF und DCM ausgewählt ist.

6. Verfahren nach Anspruch 1, wobei reduktive Spaltung des Verbindungs der Formel II mit NaBH₄/AlCl₃ durchgeführt wird.

7. Verfahren nach Anspruch 6, wobei das molaren Verhältnis von AlCl₃ und NaBH₄ gegenüber dem Ausgangsmaterial 3:1 bis 8:1, vorzugsweise 4:1 bis 7:1 fur jedes Reagenz ist.

8. Verfahren nach Anspruch 1, wobei die vorliegenden Schritten enthalten sind:
a) Behandeln der Verbindung der Formel III mit *N*-CBZ-L-valin der Formel IV, in einem organischen Lösungsmittel und in Gegenwart eines Kupplungsmittels oder eines Dehydratisierungsmittels; 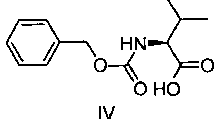
b) Entschützen der Amino-schutzgruppe carbobenzyloxygruppen und hydroxyl- geschützt benzylgruppen der erhaltenen Verbindung der Formel V Valganciclovir zu erhalten; und 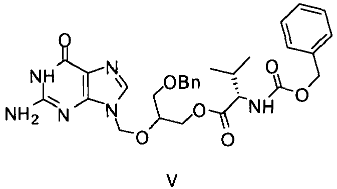
c) gegebenfalls die Umwandlung des Valganciclovirs in ein pharmazeutische annehmbare Salz davon.

9. Verfahren nach Anspruch 8, wobei das in Schritt (c) verwendete Lösungsmittel DMF oder DMSO, vorzugsweise DMSO ist.

10. Eine Verbindung der Formel II, das chemisch als 2-amino-9-((2-phenyl-1,3-dioxan-5-yloxy)methyl)-1*H*-purin-6(9*H*)-one bezeichnet

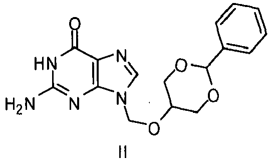

11. Verwendung von Verbindung der Formel II zur Herstellung von Valganciclovir oder pharmazeutische annehmbare Salze davon.

## Revendications

1. Un procédé pour la préparation de Valganciclovir et de ses sels pharmaceutiquement acceptables, qui comprend les étapes suivantes:
a) le traitement du Ganciclovir avec du benzaldéhyde ou du diméthyl acétal de benzaldéhyde pour obtenir la 2-amino-9-((2-phényl-1,3-dioxan-5-yloxy)méthyl)-1*H*-purin-6(9*H*)-one de Formule II; 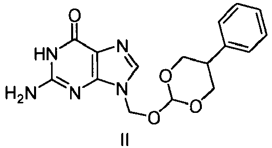
b) le clivage réducteur du composé de Formule II pour obtenir la 2-amino-9-((1-(benzyloxy)-3-hydroxypropane-2-yloxy)méthyl)-1*H*-purin-6(9*H*)-one de Formule III dans un solvant organique.

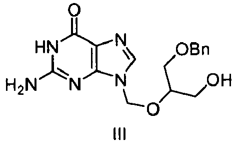

2. Le procédé selon la revendication 1, dans lequel la réaction du Ganciclovir avec le benzaldéhyde ou le diméthyl acétal de benzaldéhyde est conduite dans un solvant organique polaire aprotique et en présence d'un catalyseur acide.

3. Le procédé selon la revendication 2, dans lequel ledit solvant organique polaire aprotique est le DMF ou le DMSO, de préférence le DMSO.

4. Le procédé selon la revendication 2, dans lequel le catalyseur acide est sélectionné parmi le groupe constitué de l'acide *p*-toluène sulfonique, l'acide sulfurique, l'acide sulfonique de camphre, le chlorure de zinc et le chlorure d'étain, de préférence l'acide *p*-toluène sulfonique.

5. Le procédé selon la revendication 1, dans lequel le clivage réducteur de l'étape b) est effectué dans un solvant organique polaire sélectionné parmi THF et un mélange de THF et de DCM.

6. Le procédé selon la revendication 1, dans lequel le clivage réducteur du composé de Formule II est effectué avec NaBH₄/AlCl₃.

7. Le procédé selon la revendication 6, dans lequel le rapport molaire de AlCl₃ et de NaBH₄ par rapport au produit de départ est de 3:1 à 8:1, de préférence de 4:1 à 7:1 pour chaque réactif.

8. Le procédé selon la revendication 1, où le procédé comprend en outre les étapes:
c) le traitement du composé de Formule III avec N-CBZ-L-valine de Formule IV, dans un solvant organique et en présence d'un agent de couplage ou d'un agent déshydratant; 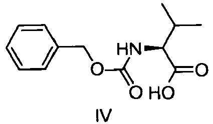
d) la déprotection du groupement aminé protégé par le carbobenzyloxy groupe et du groupement hydroxyle protégé par le benzyle groupe du composé obtenu de Formule V pour obtenir Valganciclovir; et 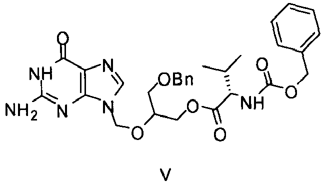
e) optionnellement, la conversion du Valganciclovir en un sel pharmaceutiquement acceptable de celui-ci.

9. Le procédé selon la revendication 8, dans lequel le solvant utilisé à l'étape c) est le DMF ou le DMSO, de préférence le DMSO.

10. Un composé de Formule II, qui est chimiquement désigné comme la 2-amino-9-((2-phényl-1,3-dioxan-5-yloxy)méthyl)-1*H*-purin-6(9*H*)-one.

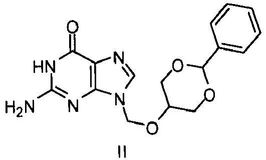

11. Utilisation du composé de Formule II pour la préparation du Valganciclovir ou des sels pharmaceutiquement acceptables de celui-ci.
